# FASCICULE DE BREVET EUROPEEN

(11) **EP 0 584 352 B1**
(45) Date de publication et mention de la délivrance du brevet: **30.07.1997**
(21) Numéro de dépôt: 93918754.8
(22) Date de dépôt: 11.03.1993
(51) Int. Cl.: A61F 2/06

(54) **ENDOPROTHESE EXPANSIBLE POUR ORGANE TUBULAIRE HUMAIN OU ANIMAL**
EXPANDIERBARE ENDOPROTHESEN FÜR MENSCHLICHE ODER TIERISCH ROHRFÖRMIGE ORGANE
EXPANSIBLE ENDOPROSTHESIS FOR HUMAN OR ANIMAL TUBULAR ORGANS

(30) Priorité: 12.03.1992 FR 9202971
(43) Date de publication de la demande: 02.03.1994
(73) Titulaire: LABORATOIRE PEROUSE IMPLANT, 60540 Bornel (FR)
(72) Inventeur: RICHARD, Thierry, F-75013 Paris (FR); PEROUSE, Eric, F-95290 L'Isle-Adam (FR)
(74) Mandataire: Jacobson, Claude
(86) Numéro de dépôt international: FR9300246
(87) Numéro de publication internationale: WO9317636

(56) Documents cités:
- EP-A- 0 364 420
- EP-A- 0 408 245
- EP-A- 0 423 916
- DE-A- 3 918 736
- DE-U- 9 001 160
- FR-A- 2 657 261
- GB-A- 2 189 150
- US-A- 4 447 222
- US-A- 4 878 906
- US-A- 4 955 859
- US-A- 4 990 155

## Description

La présente invention est relative à une endoprothèse expansible pour organe tubulaire humain ou animal, comprenant un treillis expansible de forme générale cylindrique. Elle s'applique en particulier au traitement des anévrismes et aux dilatations par voie endoluminale.

Les endoprothèses de ce type sont généralement constituées d'un simple treillis métallique dilatable au moyen d'un ballonnet, ou autoexpansible. Après une dilatation transluminale, elles sont introduites, au moyen d'une sonde, par voie endoluminale, puis dilatées ou libérées.

Ces endoprothèses connues ne sont pas totalement satisfaisantes, car les tissus pénètrent dans les mailles du treillis et sont traumatisées, et, en outre, l'endoprothèse crée des turbulences dans le flux sanguin.

De plus, ces endoprothèses ne peuvent pas être utilisées pour le traitement des anévrismes, ou plus généralement pour relier deux tronçons sains d'un organe tubulaire tel qu'un vaisseau, puisqu'elles ne sont pas étanches aux liquides.

On connaît notamment le document DE-A-39 18 736 qui décrit une endoprothèse comprenant un treillis qui est revêtu intérieurement d'un film de PTFE et éventuellement extérieurement d'un second film de PTFE.

Par ailleurs, le document GB-A-2.189.150 décrit une endoprothèse tubulaire expansible formée de fils fins et dont les extrémités sont évasées en cône vers l'extérieur à l'état dilaté.

L'invention a pour but de fournir une endoprothèse expansible qui élimine ces inconvénients. A cet effet, elle a pour objet une endoprothèse du type précité, caractérisée en ce que le treillis est noyé dans un film en une matière plastique ou élastomère extensible et biocompatible, ce film emplissant les mailles du treillis et recouvrant la totalité de la surface du treillis dans la partie courante de celui-ci, l'extensibilité de la matière formant le film étant suffisante pour que celui- ci puisse suivre la déformation du treillis de son état contracté à son état dilaté.

Suivant d'autres caractéristiques :
- le film est constitué d'un polymère tel qu'un polyuréthanne ou d'un caoutchouc, naturel ou synthétique;
- l'endoprothèse étant du type autoexpansible, les parties d'extrémité de l'endoprothèse sont évasées à l'état dilaté de celle-ci;
- le treillis est en acier inoxydable ou en une matière plastique relativement rigide telle que le polytétrafluoroéthylène rendue radio-opaque.

L'invention a également pour objet un outil de mise en place d'une endoprothèse autoexpansible telle que définie ci-dessus. Cet outil comprend :
- un conduit-guide pourvu à son extrémité distale d'une tulipe de logement de l'endoprothèse à l'état contracté; et
- des moyens pour ouvrir longitudinalement la tulipe.

Suivant un mode de réalisation, lesdits moyens comprennent des fils de découpe de la tulipe en plusieurs pétales, reliés à une poignée d'actionnement.

Suivant un autre mode de réalisation, lesdits moyens comprennent une ouverture longitudinale de la tulipe dont chaque bord présente une série de goussets, les goussets des deux bords étant imbriqués les uns dans les autres et étant maintenus par un cordon qui les traverse et qui est relié à une poignée d'actionnement.

Des exemples de réalisation de l'invention vont maintenant être décrits en regard du dessin annexé, sur lequel :
- la Figure 1 représente schématiquement une endoprothèse suivant l'invention à l'état rétracté;
- la Figure 2 représente schématiquement la même endoprothèse à l'état dilaté;
- la Figure 3 représente à échelle très agrandie, en perspective, un outil de mise en place d'une endoprothèse autoexpansible suivant l'invention;
- la Figure 4 est une vue en coupe longitudinale de l'outil de la Figure 3;
- la Figure 5 est une vue prise en coupe suivant la ligne V-V de la Figure 4;
- la Figure 6 illustre l'utilisation de l'outil des Figures 3 à 5;
- la Figure 7 illustre la dilatation correspondante de l'endoprothèse; et
- la Figure 8 représente schématiquement, à échelle très agrandie et en perspective, un autre outil de mise en place d'une endoprothèse autoexpansible suivant l'invention.

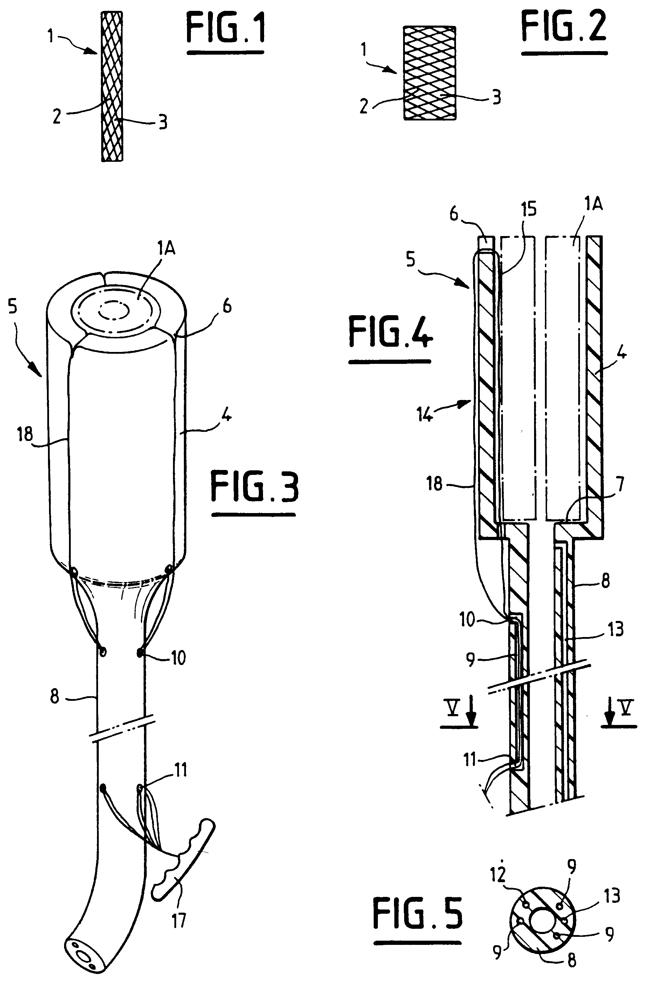

L'endoprothèse l représentée aux Figures 1 et 2 est constituée d'un treillis tubulaire 2 noyé dans un film 3.

Le treillis 2 est constitué d'acier inoxydable de qualité biocompatible. Il peut être réalisé par tissage ou tricotage d'un fil, déploiement axial d'un tube, ou par toute autre technique appropriée. Il est plastiquement déformable, c'est-à-dire qu'il possède une première forme stable de petit diamètre, représentée à la Figure 1, dans laquelle les mailles forment des losanges allongés parallèlement à son axe, et une seconde forme stable de diamètre très agrandi et de plus courte longueur, représentée à la Figure 2, dans laquelle les mailles forment des losanges allongés dans le sens circonférentiel.

Le treillis 2 est entièrement noyé dans un film 3 d'une matière extensible et étanche aux liquides qui en emplit les mailles. L'extensibilité de cette matière est suffisante pour que le film 3 puisse suivre la déformation du treillis 2 de son état contracté à son état dilaté sans déchirure ni décollement, malgré la déformation des mailles du treillis. Des matières appropriées sont un élastomère biocompatible, qui peut être un caoutchouc naturel ou synthétique, ou bien un polymère biocompatible tel qu'un polyuréthanne.

L'enrobage du treillis 2 par le film 3 peut être obtenu par des techniques de co-extrusion ou de trempage, après dégraissage du métal et son traitement par une substance primaire d'adhérence.

On obtient donc, à l'état dilaté (Figure 2), un tronçon tubulaire étanche aux liquides qui peut être utilisé comme endoprothèse ou "stent" après une dilatation transluminale. Cette endoprothèse ne traumatise pas les tissus et ne crée pratiquement pas de turbulences dans le flux sanguin, puisque les tissus et le sang sont au contact d'un surface pratiquement lisse en élastomère ou en polymère.

Du fait de son étanchéité, l'endoprothèse peut être utilisée pour traiter par voie endoluminale un anévrisme, en la faisant ponter l'anévrisme, chacune de ses extrémités s'appliquant radialement contre la paroi intérieure d'un tronçon d'artère sain adjacent à l'anévrisme.

Dans un autre mode de réalisation, illustré aux Figures 3 à 7, le treillis 2 de l'endoprothèse 1A est autoexpansible, ce qui s'obtient de façon classique par utilisation d'un acier inoxydable ayant des propriétés de ressort.

Pour mettre en place l'endoprothèse 1A, on la comprime radialement jusqu'à sa configuration de la Figure 1, qui n'est pas stable, et on l'introduit dans la tulipe d'extrémité 4 d'un outil 5 représenté sur les Figures 3 à 5.

L'extrémité distale de la tulipe 4 est ouverte et présente trois échancrures 6 à 120° les unes des autres. Son extrémité proximale forme un épaulement intérieur 7 d'où part un conduit de guidage 8. Dans le plan de chaque échancrure 6, un canal 9 formé dans l'épaisseur de paroi du conduit 8 débouche à l'extérieur par des orifices radiaux 10, 11, d'une part près de l'épaulement 7, d'autre part près de l'extrémité proximale du conduit 8.

On peut également prévoir dans l'épaisseur de paroi du conduit 8, comme représenté, des canaux longitudinaux 12, 13 d'injection de fluides, qui partent de l'extrémité proximale de ce conduit et débouchent dans la lumière intérieure du conduit 8 près de l'épaulement 7.

Dans chacun des trois plans précités, un fil souple 14 passe dans l'échancrure 6. Un brin intérieur 15 de ce fil longe la paroi intérieure de la tulipe 4, traverse un orifice 16 prévu dans l'épaulement 7, pénètre dans l'orifice 10, s'étend le long du canal 9, sort par l'orifice 11 et rejoint une poignée d'actionnement 17 (Figure 3). Un brin extérieur 18 du fil 14 longe la paroi extérieure de la tulipe, suit le même trajet 19, 11 que le brin 15, et rejoint également la poignée 17. Celle-ci est donc reliée à six brins de fils, et les trois brins intérieurs 15 sont plaqués contre la paroi intérieure de la tulipe par la tendance à l'expansion de l'endoprothèse 1A.

Pour l'utilisation de l'endoprothèse, après une dilatation transluminale ou pour traiter un anévrisme, l'outil 5 est enfilé sur un guide, introduit à travers la peau et conduit par voie endoluminale jusqu'à l'emplacement désiré.

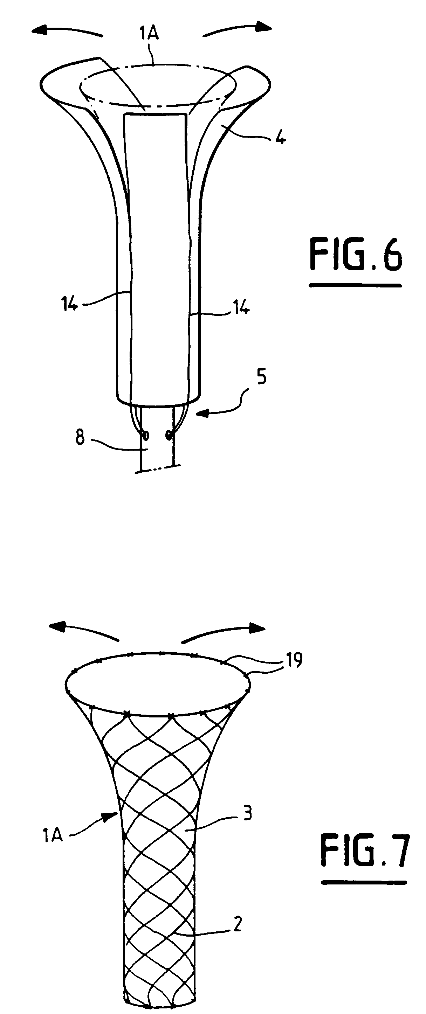

L'opérateur tire alors sur la poignée 17. Celle-ci met les trois fils 15 en tension, et ces fils découpent chacun la tulipe 4 suivant une génératrice. La tulipe libère donc progressivement l'endoprothèse, laquelle se dilate d'elle-même, comme illustré sur la Figure 6. Lorsque la tulipe est entièrement ouverte, on retire l'outil par traction sur le conduit 8.

A l'état dilaté (Figure 7), on constate que les deux extrémités de l'endoprothèse se sont évasées d'elles-mêmes, ce qui procure deux effets avantageux : d'une part, l'étanchéité entre l'endoprothèse et l'artère est renforcée, et d'autre part, les extrémités 19 des fils du treillis 2 dépassent légèrement du film 3 et constituent autant de pointes d'accrochage de l'endoprothèse dans l'artère. La stabilité du positionnement de l'endoprothèse est ainsi assurée.

D'autres matériaux peuvent être utilisés pour constituer le treillis 2. Par exemple, pour réaliser une endoprothèse autoexpansible, on peut utiliser du fil d'un polymère relativement rigide et à propriétés de ressort tel que le polytétrafluoroéthylène (PTFE), rendu radio-opaque.

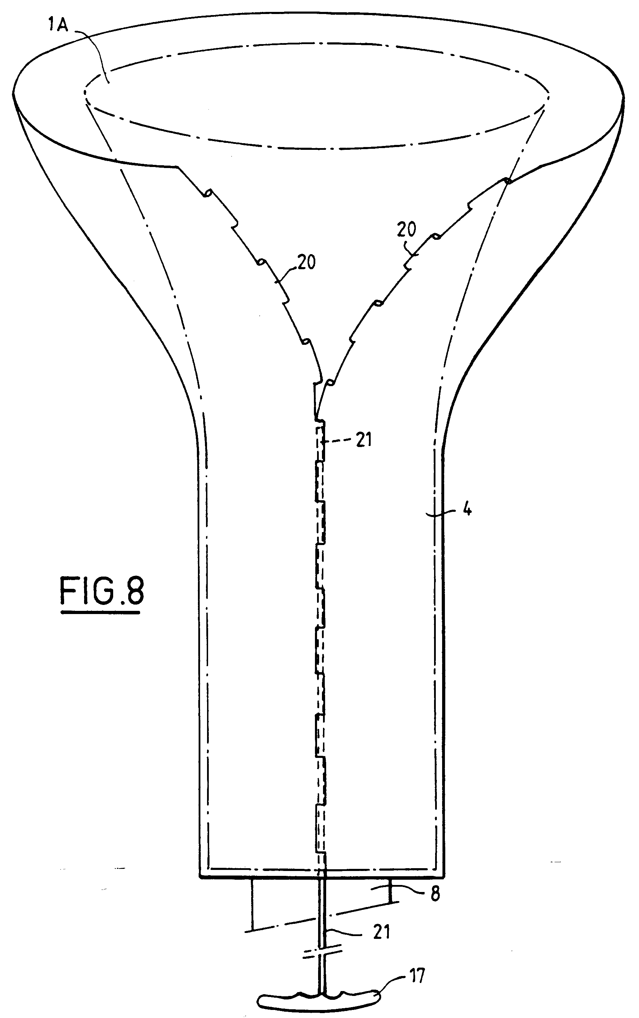

On a représenté schématiquement à la Figure 8 un autre mode de réalisation de l'outil 5, qui diffère de celui décrit plus haut par les moyens d'ouverture longitudinale de la tulipe.

En effet, la tulipe est fendue longitudinalement sur toute sa hauteur. Chaque bord de la fente comporte une série de goussets cylindriques 20 en saillie. Lorsque la tulipe est dans son état cylindrique fermé, et maintient une endoprothèse autoexpansible 1A à l'état contracté, les goussets 20 des deux bords s'interpénètrent, et l'ensemble est maintenu par un cordon 21 qui traverse tous les goussets et est relié, à son extrémité proximale, à la poignée d'actionnement 17.

La libération de l'endoprothèse s'effectue par simple traction sur la poignée 17.

## Revendications

1. Endoprothèse expansible pour organe tubulaire humain ou animal, comprenant un treillis expansible (2; 2A) de forme générale cylindrique, caractérisée en ce que le treillis est noyé dans un film (3) en une matière plastique ou élastomère extensible et biocompatible, ce film emplissant les mailles du treillis et recouvrant la totalité de la surface du treillis (2) dans la partie courante de celui-ci, l'extensibilité de la matière formant le film étant suffisante pour que celui-ci puisse suivre la déformation du treillis (2) de son état contracté à son état dilaté.

2. Endoprothèse suivant la revendication 1, caractérisée en ce que le film (3) est constitué d'un polymère tel qu'un polyuréthanne ou d'un caoutchouc naturel ou synthétique.

3. Endoprothèse suivant la revendication 1 ou 2, du type autoexpansible, caractérisée en ce que les parties d'extrémité de l'endoprothèse (1A) sont évasées à l'état dilaté de celle-ci.

4. Endoprothèse suivant l'une quelconque des revendications 1 à 3, caractérisé en ce que le treillis (2) est en acier inoxydable.

5. Endoprothèse suivant l'une quelconque des revendications 1 à 3, caractérisée en ce que le treillis (2) est en une matière plastique relativement rigide telle que le polytétrafluoroéthylène, rendue radio-opaque.

6. Outil de mise en place d'une endoprothèse autoexpansible, caractérisé en ce qu'il comprend :
- un conduit-guide (8) pourvu à son extrémité distale d'une tulipe (4) de logement de l'endoprothèse (1A) à l'état contracté ; et
- des moyens (14, 17; 20, 21, 17) pour ouvrir longitudinalement la tulipe.

7. Outil suivant la revendication 6, caractérisé en ce que lesdits moyens (14, 17) comprennent des fils de découpe de la tulipe (4) en plusieurs pétales, reliés à un poignée d'actionnement (17).

8. Outil suivant la revendication 6, caractérisé en ce que lesdits moyens (20, 21, 17) comprennent une ouverture longitudinale de la tulipe dont chaque bord présente une série de goussets (20), les goussets des deux bords étant imbriqués les uns dans les autres et étant maintenus par un cordon (21) qui les traverse et qui est relié à une poignée d'actionnement (17).

## Claims

1. Expansible endoprosthesis for human or animal tubular organs, comprising an expansible net (2; 2A) of generally cylindrical shape, characterised in that the net is embedded in a film (3) of a extensible and biocompatible plastic or elastomer material, this film filling the meshes of the net and covering the entirety of the surface of the net (2) in the operating part of the latter, the extensibility of the material forming the film being sufficient for the latter to be able to follow the deformation of the net (2) from its contracted state to its dilated state.

2. Endoprosthesis according to claim 1, characterised in that the film (3) consists of a polymer such as a polyurethane or a natural or synthetic rubber.

3. Endoprosthesis according to claim 1 or claim 2 of the self-expanding type, characterised in that the end parts of the endoprosthesis (1A) are flared in the dilated state of the latter.

4. Endoprosthesis according to any one of claims 1 to 3, characterised in that the net (2) is made of stainless steel.

5. Endoprosthesis according to any one of claims 1 to 3, characterised in that the net (2) is made of a relatively rigid plastic material such as polytetrafluoroethylene, rendered radio-opaque.

6. Tool for positioning a self-expanding endoprosthesis, characterised in that it comprises:
- a guide conduit (8) provided at its distal end with a tulip (4) for housing the endoprosthesis (1A) in the contracted state, and
- means (14, 17; 20, 21,17) for opening the tulip longitudinally.

7. Tool according to claim 6, characterised in that the said means (14, 17) comprise threads for cutting the tulip (4) into several petals, connected to a operating handle (17).

8. Tool according to claim 6, characterised in that the said means (20, 21, 17) comprise a longitudinal opening in the tulip, each edge of which has a series of gussets (20), the gussets of the two edges being interlaced with one another and being held by a cord (21) which traverses the latter and is connected to an operating handle (17).

## Patentansprüche

1. Expandierbare Endoprothese für menschliche oder tierische rohrförmige Organe, die ein expandierbares Gitter (2; 2A) in allgemeiner Zylinderform umfaßt, dadurch gekennzeichnet, daß das Gitter in einen Film (3) aus einem dehnbaren und bioverträglichen Plastik- oder Elastomermaterial eingelassen ist, wobei der Film die Maschen des Gitters auffüllt und die gesamte Oberfläche des Gitters (2) im laufenden Teil desselben bedeckt, wobei die Dehnbarkeit des Materials, das den Film bildet, ausreichend ist, um dem Film zu ermöglichen, der Verformung des Gitters (2) von dessen zusammengezogenen Zustand zu dessen ausgedehnten Zustand zu folgen.

2. Endoprothese nach Anspruch 1, dadurch gekennzeichnet, daß der Film (3) aus einem Polymer wie einem Polyurethan oder einem Naturkautschuk oder einem Kunstkautschuk besteht.

3. Endoprothese nach Anspruch 1 oder 2, der selbstexpandierbaren Art, dadurch gekennzeichnet, daß die Endteile der Endoprothese (1A) im ausgedehnten Zustand derselben konisch erweitert sind.

4. Endoprothese nach einem der Ansprüche 1 bis 3, dadurch gekennzeichnet, daß das Gitter (2) aus rostfreiem Stahl besteht.

5. Endoprothese nach einem der Ansprüche 1 bis 3, dadurch gekennzeichnet, daß das Gitter (2) aus einem relativ steifen Plastikmaterial besteht, wie Polytetrafluorethylen, das strahlenundurchlässig gemacht wird.

6. Werkzeug zum Positionieren einer selbstexpandierbaren Endoprothese, dadurch gekennzeichnet, daß es folgendes umfaßt:
- einen Führungskanal (8), der an seinem distalen Ende mit einer Tulpe (4) für die Aufnahme der Endoprothese (1A) im zusammengezogenen Zustand versehen ist; und
- Mittel (14, 17; 20, 21, 17) zum längsseitigen Öffnen der Tulpe.

7. Werkzeug nach Anspruch 6, dadurch gekennzeichnet, daß die Mittel (14, 17) Fäden zum Schneiden der Tulpe (4) in mehrere blütenblattförmige Teile umfassen, wobei die Fäden mit einem Betätigungsgriff verbunden sind.

8. Werkzeug nach Anspruch 6, dadurch gekennzeichnet, daß die Mittel (20, 21, 17) eine Längsöffnung der Tulpe umfassen, bei der jeder Rand eine Reihe von Lappen (20) aufweist, wobei die Lappen der zwei Ränder miteinander verschachtelt sind und durch eine Schnur (21) gehalten werden, die durch sie hindurch verläuft und die mit einem Betätigungsgriff (17) verbunden ist.
